# EUROPEAN PATENT SPECIFICATION

(11) **EP 3 793 779 B1**
(45) Date of publication and mention of the grant of the patent: **27.04.2022**
(21) Application number: 19743006.9
(22) Date of filing: 16.05.2019
(51) Int. Cl.: B25J 9/00, B25J 13/02, G06F 3/01, A61H 1/02, A61F 2/58, A61F 5/01

(54) **EXOSKELETON GLOVE**
EXOSKELETTHANDSCHUH
GANT D'EXOSQUELETTE

(30) Priority: 16.05.2018 NL 2020941
(43) Date of publication of application: 24.03.2021
(73) Proprietor: ADJUVO MOTION B.V., 2629 JD Delft (NL)
(72) Inventor: LUIJTEN, Johannes, 2600 AA Delft (NL); DEN BUTTER, Gijs, 2600 AA Delft (NL); LAMMERS, Max, 2600 AA Delft (NL)
(74) Representative: Van Breda, Jacobus
(86) International application number: PCT/NL2019/050286
(87) International publication number: WO 2019/221604

(56) References cited:
- EP-A2- 2 345 951
- WO-A1-99/21478
- US-A1- 2013 219 585
- US-A1- 2017 071 272

## Description

The invention relates to an exoskeleton glove provided with an individual linkage mechanism for at least a finger or a thumb, wherein the linkage mechanism comprises a series of linkages concatenated through joints, a first linkage being attached to the glove, and a last linkage at a farthest end from the glove being provided with a thimble.

WO2018/021909 teaches such an exoskeleton glove.

EP-A-2 345 951 and US2013/0219585 each disclose an exoskeleton glove provided with an individual linkage mechanism for at least a finger or a thumb, wherein the linkage mechanism comprises a series of linkages concatenated through joints, a first linkage being attached to the glove, and a last linkage at a farthest end from the glove being provided with a thimble, wherein a cable guided within or alongside the linkage mechanism connects the last linkage and/or the thimble to the first linkage so as to transfer a force through the cable acting on the last linkage and/or the thimble.

WO99/21478 discloses an exoskeleton device for measuring positions of links and angles of joints of an animate body, where the body comprises a plurality of links joined by intervening joints. The device is affixed at a first mobile terminus of said animate body, and a second fixed terminus, having device links displaced from animate links, where the device links are connected by device joints and having sensor means for measuring the angle of the device joints. Using the signals from the sensor means, one can determine the position of the terminal device link, and based on knowledge of the animate body structure calculate the animate angle joints.

The invention has as an objective to modify the known exoskeleton glove to enable a user to interact with virtual objects in a way similar to real world objects.

It is a further object of the invention to provide an exoskeleton glove which is suitable for providing force and tactile feedback to the fingers of a user of the glove.

It is still a further object of the invention to keep weight of the exoskeleton glove down and to make it possible to use the glove without features that require external powering.

In order to promote one or more of the above-mentioned objectives the exoskeleton glove of the invention has the features of one or more of the appended claims.

According to invention a spring-loaded pulley for winding or unwinding the cable is provided at or near the first linkage, wherein the pulley is connected with a magnetic brake. With the cable a mechanical force feedback can be provided to the finger of the user that corresponds with the linkage mechanism. The spring-loaded pulley enables that during the motion of the finger, a continuous tension can be provided on the cable. By applying a pulley it is further realized that a linear translation is converted into a rotation for retracting the cable. The magnetic brake makes possible that at a moment when a virtual object connects to a virtual representation of the finger, the brake provides a frictional force to the pulley. This frictional force is transmitted via the cable to the tip of the finger, i.e. to the last linkage and/or the thimble.

Preferably the magnetic brake is drivable by a variable voltage. By regulating this voltage a variety of effects can be communicated to the user's finger such as the shape and the density of a virtual object.

By arranging that the glove is battery-powered, the glove will be very user-friendly without need to connect to an external power source.

The exoskeleton glove of the invention is suitably embodied such that the cable is guided over a guide wheel in the linkage mechanism which is positioned distant from the glove.

In another aspect of the invention the linkage mechanism is provided with a vibro-haptic feedback motor. With this feedback motor additional information on the virtual object can be communicated to the user's finger, such as hardness, roughness or for instance simulation of a point of entry in a virtual object. For the same purpose it is also possible that at least one actuator acting on the cable is provided.

The invention will hereinafter be further elucidated with reference to the drawing of an exemplary embodiment of an exoskeleton glove according to the invention and its use that is not limiting as to the appended claims.

In the drawing:
- figure 1A and figure 1B show in different perspectives a glove of an exoskeleton glove according to the prior art provided on a hand of the user;
- figure 2A and 2B show the individual linkage mechanisms of the thumb and the fingers of the prior art glove in different perspectives;
- figure 3 shows a linkage mechanism for a finger of an exoskeleton glove of the invention;
- figure 4 shows a sectional view of the linkage mechanism shown in figure 3.

Whenever in the figures the same reference numerals are applied, these numerals refer to the same parts.

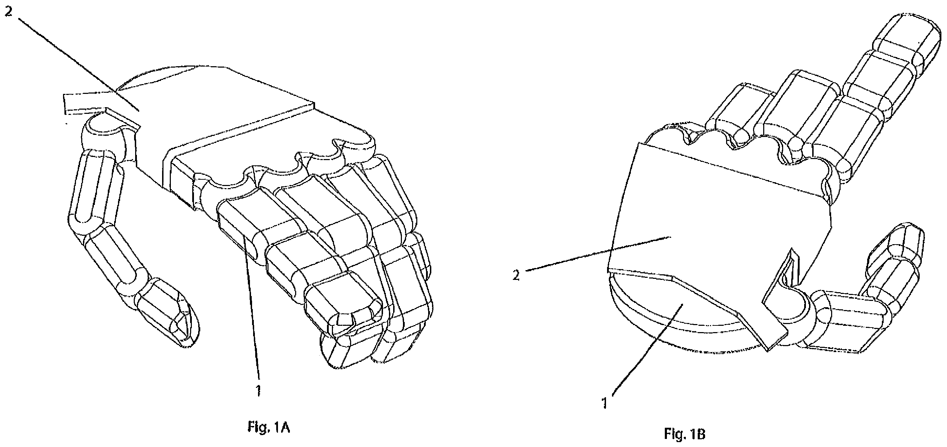

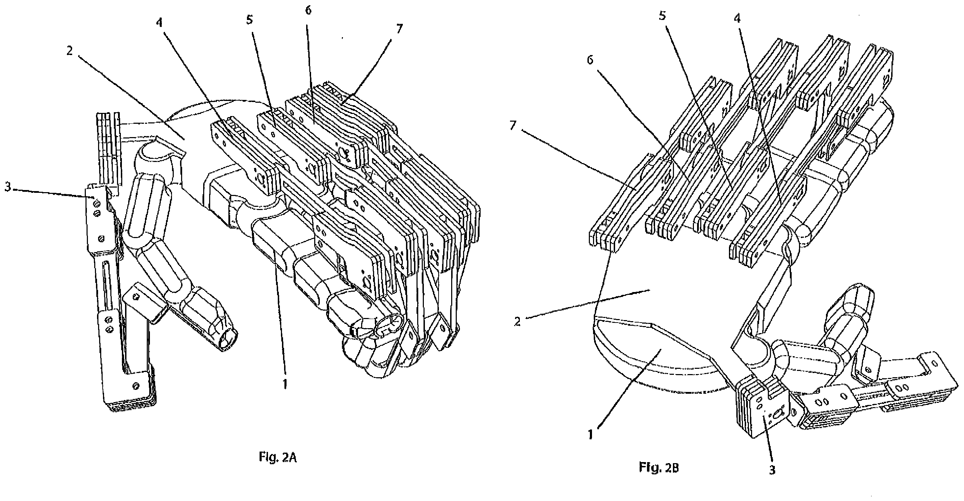

Figure 1A and figure 1B show in different perspectives a user's hand 1 provided with a glove 2 of an exoskeleton glove of the prior art which is shown in more detail in figure 2A and figure 2B.

In figure 2A and figure 2B it is shown that the exoskeleton glove is provided with an individual linkage mechanism for each finger and for the thumb. It shows a linkage mechanism 3 for the thumb, and further there are a linkage mechanism 4 for the index finger, a linkage mechanism 5 for the middle finger, a linkage mechanism 6 for the ring finger, and a linkage mechanism 7 for the small finger. The linkage mechanism 3 for the thumb is a bit more complicated than the linkage mechanisms for the fingers. The construction of the linkage mechanisms for the fingers is however for each finger the same. Accordingly the linkage mechanism comprises in any case a series of linkages 8, 11, 15, 19 concatenated through joints 10, 17, 20, wherein a first linkage is attached to the glove 2, and a last linkage 19 farthest from the glove is provided with a thimble 18.

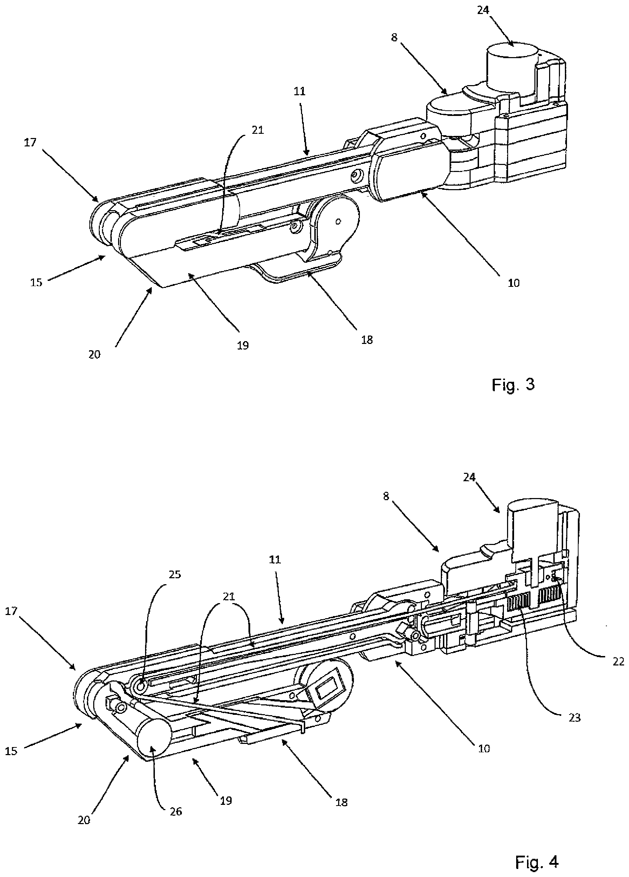

Figure 3 and figure 4 shows the linkage mechanism for a single finger as forming part of the exoskeleton glove of the invention. It is noted that although not shown the linkage mechanism for the thumb of the exoskeleton glove of the invention is arranged similarly, so that a separate illustration thereof is superfluous. Figure 3 shows the linkage mechanism in full view, whereas figure 4 shows the linkage mechanism in a sectional view through a longitudinal axis of the mechanism.

Figures 3 and 4 show that the linkage mechanism comprises several sections that are interconnected with joints to enable changing their mutual angular orientation. The linkage mechanism comprises a first linkage 8 which is intended to be attached to the glove 2 (see figures 2A/B), a second linkage 11 connected to the first linkage 8 through a first joint 9, a third linkage 15 connected to the second linkage 11 through a second joint 17, and a fourth linkage 19 connected to the third linkage 15 through a third joint 20, wherein the fourth linkage 19 is provided with a thimble 18. The thimble 18 is evidently at a farthest end from the glove 2.

Figure 3 and more clearly figure 4 further show that a cable 21 is guided within (or alongside) the linkage mechanism that connects the last linkage 19 distant from the glove 2 and/or the thimble 18 to the first linkage 8 so as to transfer a force through the cable 21 acting on the last linkage 19 and/or the thimble 18.

At or near the first linkage 8 a spring-loaded pulley 22 is provided for winding or unwinding the cable 21. The spring for the pulley 22 is carrying reference 23.

The pulley is 22 connected with a magnetic brake 24, which is desirably drivable by a variable voltage, for instance by pulse width modulation PWM, which is known per se to the skilled person.

Preferably one thing and another is battery-powered. This is not shown in the drawing but is clear for the skilled person and requires no further elucidation.

Figure 4 further shows that the cable 21 is guided over a guide wheel 25 in the linkage mechanism which is positioned distant from the glove 2.

Finally figure 4 shows that the linkage mechanism is provided with a vibro-haptic feedback motor 26.

Although the invention has been discussed in the foregoing with reference to an exemplary embodiment of a part of the exoskeleton glove of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

## Claims

1. Exoskeleton glove (2) provided with an individual linkage mechanism (4, 5, 6, 7) for at least a finger or a thumb, wherein the linkage mechanism comprises a series of linkages (8, 11, 15, 19) concatenated through joints (10, 17, 20), a first linkage being attached to the glove (2), and a last linkage (19) at a farthest end from the glove being provided with a thimble (18), wherein a cable (21) guided within or alongside the linkage mechanism connects the last linkage (19) and/or the thimble (18) to the first linkage (8) so as to transfer a force through the cable (21) acting on the last linkage (19) and/or the thimble (18, **characterized in that** at or near the first linkage (8) a spring-loaded pulley (22) is provided for winding or unwinding the cable (21), and that the pulley (22) is connected with a magnetic brake (24).

2. Exoskeleton glove according to claim 1, **characterized in that** the magnetic brake (24) is drivable by a variable voltage.

3. Exoskeleton glove according to any one of the previous claims 1 - 2, **characterized in that** the glove (2) is battery-powered.

4. Exoskeleton glove according to any one of the previous claims 1 - 3, **characterized in that** the cable (21) is guided over a guide wheel (25) in the linkage mechanism which is positioned distant from the glove (2).

5. Exoskeleton glove according to any one of the previous claims 1 - 4, **characterized in that** the linkage mechanism is provided with a vibro-haptic feedback motor (26).

6. Exoskeleton glove according to any one of the previous claims 1 - 5, **characterized in that** at least one actuator acting on the cable (21) is provided.

## Patentansprüche

1. Exoskelett-Handschuh (2), der mit einem individuellen Verbindungsmechanismus (4, 5, 6, 7) für mindestens einen Finger oder einen Daumen versehen ist, wobei der Verbindungsmechanismus eine Folge von Verbindungen (8, 11, 15, 19) aufweist, die miteinander durch Gelenke (10, 17, 20) gekoppelt sind, wobei eine erste Verbindung an dem Handschuh (2) befestigt ist und eine letzte Verbindung (19) an einem am weitesten vom Handschuh entfernten Ende mit einem Fingerhut (18) versehen ist, wobei ein Seil (21), das innerhalb oder neben dem Verbindungsmechanismus geführt ist, die letzte Verbindung (19) und/oder den Fingerhut (18) mit der ersten Verbindung (8) verbindet, um eine auf die letzte Verbindung (19) wirkende Kraft über das Seil (21) und/oder den Fingerhut (18) zu übertragen, **dadurch gekennzeichnet, dass** an oder nahe der ersten Verbindung (8) eine federbelastete Rolle (22) zum Auf- oder Abwickeln des Seils (21) vorgesehen ist, und dass die Rolle (22) mit eine Magnetbremse (24) verbunden ist.

2. Exoskelett-Handschuh nach Anspruch 1, **dadurch gekennzeichnet, dass** die Magnetbremse (24) durch eine variable Spannung ansteuerbar ist.

3. Exoskelett-Handschuh nach einem der vorhergehenden Ansprüche 1 bis 2, **dadurch gekennzeichnet, dass** der Handschuh (2) batteriebetrieben ist.

4. Exoskelett-Handschuh nach einem der vorhergehenden Ansprüche 1 bis 3, **dadurch gekennzeichnet, dass** das Seil (21) über ein Umlenkrad (25) in dem Verbindungsmechanismus geführt ist, welches in einer Entfernung zum Handschuh (2) positioniert ist.

5. Exoskelett-Handschuh nach einem der vorhergehenden Ansprüche 1 bis 4, **dadurch gekennzeichnet, dass** der Verbindungsmechanismus mit einem vibro-haptischen Rückkopplungsmotor (26) versehen ist.

6. Exoskelett-Handschuh nach einem der vorhergehenden Ansprüche 1 - 5, **dadurch gekennzeichnet, dass** mindestens ein auf das Seil (21) wirkender Aktuator vorgesehen ist.

## Revendications

1. Gant d'exosquelette (2) prévu avec un mécanisme de liaison individuel (4, 5, 6, 7) pour au moins un doigt ou un pouce, dans lequel le mécanisme de liaison comprend une série de liaisons (8, 11, 15, 19) reliées par le biais d'articulations (10, 17, 20), une première liaison étant fixée au gant (2), et une dernière liaison (19), au niveau de l'extrémité la plus éloignée du gant, étant prévue avec un dé (18), dans lequel un câble (21) guidé dans ou le long du mécanisme de liaison raccorde la dernière liaison (19) et/ou le dé (18) à la première liaison (8) afin de transférer une force par le biais du câble (21) qui agit sur la dernière liaison (19) et/ou le dé (18), **caractérisé en ce qu'**au niveau de ou à proximité de la première liaison (8), une poulie à ressort (22) est prévue pour enrouler ou dérouler le câble (21), et **en ce que** la poulie (22) est raccordée avec un frein magnétique (24).

2. Gant d'exosquelette selon la revendication 1, **caractérisé en ce que** le frein magnétique (24) peut être entraîné par une tension variable.

3. Gant d'exosquelette selon l'une quelconque des revendications 1 à 2, le gant (2) étant **caractérisé en ce qu'**il est alimenté par batterie.

4. Gant d'exosquelette selon l'une quelconque des revendications 1 à 3, **caractérisé en ce que** le câble (21) est guidé sur une roue de guidage (25) dans le mécanisme de liaison qui est positionné à distance du gant (2).

5. Gant d'exosquelette selon l'une quelconque des revendications 1 à 4, **caractérisé en ce que** le mécanisme de liaison est prévu avec un moteur de rétroaction vibro-haptique (26).

6. Gant d'exosquelette selon l'une quelconque des revendications 1 à 5, **caractérisé en ce qu'**au moins un actionneur agissant sur le câble (21) est prévu.
